# FASCICULE DE BREVET EUROPEEN

(11) **EP 3 648 768 B1**
(45) Date de publication et mention de la délivrance du brevet: **07.06.2023**
(21) Numéro de dépôt: 18750243.0
(22) Date de dépôt: 04.07.2018
(51) Int. Cl.: A61K 31/53, A61P 35/00, A61K 45/06, A61K 31/44

(54) **IMÉGLIMINE POUR LA PRÉVENTION ET/OU LE TRAITEMENT DU CARCINOME HÉPATOCELLULAIRE**
IMEGLIMIN ZUR PRÄVENTION UND/ODER BEHANDLUNG VON LEBERZELLKARZINOMEN
IMEGLIMIN FOR PREVENTING AND/OR TREATING HEPATOCELLULAR CARCINOMA

(30) Priorité: 05.07.2017 FR 1700713
(43) Date de publication de la demande: 13.05.2020
(73) Titulaire: ERICARE, 31500 Toulouse (FR)
(72) Inventeur: JANIN, Eric, 31500 Toulouse (FR)
(74) Mandataire: Bringer IP
(86) Numéro de dépôt international: PCT/FR2018/000188
(87) Numéro de publication internationale: WO 2019/008239

(56) Documents cités:
- WO-A1-01/55122
- WO-A1-2011/006983
- WO-A2-2010/066901
- F. CAUCHY ET AL: "Strong antineoplastic effects of metformin in preclinical models of liver carcinogenesis", CLINICAL SCIENCE., vol. 131, no. 1, 1 novembre 2016 (2016-11-01), pages 27-36, XP055458956, GB ISSN: 0143-5221, DOI: 10.1042/CS20160438
- SUNBIN LING ET AL: "Metformin reverses multidrug resistance in human hepatocellular carcinoma Bel-7402/5-fluorouracil cells", MOLECULAR MEDICINE REPORTS, vol. 10, no. 6, 8 octobre 2014 (2014-10-08), pages 2891-2897, XP055458966, GR ISSN: 1791-2997, DOI: 10.3892/mmr.2014.2614
- V. PIRAGS ET AL: "Imeglimin, a novel glimin oral antidiabetic, exhibits a good efficacy and safety profile in type 2 diabetic patients", DIABETES, OBESITY AND METABOLISM, vol. 14, no. 9, 16 mai 2012 (2012-05-16), pages 852-858, XP055458567, ISSN: 1462-8902, DOI: 10.1111/j.1463-1326.2012.01611.x
- SWAMY SUPRITHA G ET AL: "Targeting multiple oncogenic pathways for the treatment of hepatocellular carcinoma", TARGETED ONCOLOGY, SPRINGER PARIS, PARIS, vol. 12, no. 1, 11 août 2016 (2016-08-11), pages 1-10, XP036142460, ISSN: 1776-2596, DOI: 10.1007/S11523-016-0452-7 [extrait le 2016-08-11]
- BRUIX JORDI ET AL: "Evidence-Based Diagnosis, Staging, and Treatment of Patients With Hepatocellular Carcinoma", GASTROENTEROLOGY, W.B. SAUNDERS CO, US, vol. 150, no. 4, 12 janvier 2016 (2016-01-12), pages 835-853, XP029469489, ISSN: 0016-5085, DOI: 10.1053/J.GASTRO.2015.12.041

## Description

La présente invention concerne l'utilisation de l'iméglimine ou ((6R)-(+)-4-dimethylamino- 2- imino-6-methyl-1,2,5,6-tetrahydro-1,3,5-triazine hydrochloride) de formule (I) 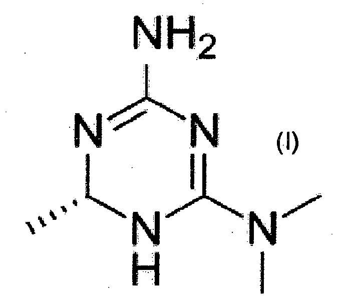 pour la prévention et/ou le traitement du carcinome hépatocellulaire.

Les cancers du foie représentent mondialement plus de 850 000 nouveaux cas par année, dont 90% pour le carcinome hépatocellulaire. Le carcinome hépatocellulaire est la troisième cause de mortalité par cancer dans le monde. L'infection chronique par le virus de l'hépatite C (VHC) ou par le virus de l'hépatite B (VHB) est la principale cause du carcinome hépatocellulaire (Nat Rev Dis Primers 2016, 2 : 16018).

L'incidence des carcinomes hépatocellulaires est plus élevée chez les diabétiques de type 2 que chez les non-diabétiques qu'ils soient ou non porteurs de maladies du foie (Curr Diab Rep 2017, 17 :20).

Le traitement du carcinome hépatocellulaire est indispensable sous peine d'une évolution fatale. Les traitements curatifs actuels sont la chirurgie avec résection de la tumeur et/ou transplantation hépatique. Cependant, lorsque le diagnostic est tardif et la maladie découverte à un stade avancé, ces traitements ne sont plus envisageables. Le traitement de référence à ce stade avancé est le sorafénib, petite molécule active par voie orale, un inhibiteur multikinase, antiangiogénique. Chez ces patients la médiane de survie est de 10,7 mois chez ceux traités par sorafénib et de 7,9 mois chez ceux recevant un placebo. Diarrhées, perte de poids, syndrome main-pied et hypophosphatémie sont les effets secondaires les plus importants dans le groupe sorafénib (New England Journal of Medicine 2008, 359: 378-90)

La metformine, biguanide normoglycémiant, est un antidiabétique oral diminuant la production hépatique de glucose. Il est le traitement de référence du diabète de type 2, le plus prescrit en première intention.

L'inhibition du complexe I mitochondrial et l'activation de l'AMP-activated protein kinase (AMPK) sont impliquées dans les effets de la metformine (Cell Metabolism 2014, 20 : 953-66).

Des études épidémiologiques, rétrospectives ou de cohortes, montrent que des diabétiques de type 2 traités par metformine présentent moins de cancers que des diabétiques traités par d'autres antidiabétiques (Diabètes Care 2009, 9 :1620-25; BMC Cancer 2011, 11:20; Gut 2013, 62 :606-15) et en particulier dans le carcinome hépatocellulaire (Am J Gastroenterol 2013, 108 : 881-91).

Des études expérimentales ont également montré que la metformine possédait des propriétés anti-tumorales innées. Dans le carcinome hépatocellulaire, *in vitro,* la metformine possède une activité inhibitrice de la croissance cellulaire sur les lignées HepG2 et HuH7. *In vivo,* sur des modèles d'hétérogreffes sous-cutanées de cellules tumorales humaines hépatiques, chez des souris immunodéprimées, la metformine montre une activité anticancéreuse. Des études cliniques sont en cours pour confirmer dans différents cancers l'activité de la metformine en association à des anticancéreux. (Gut 2013, 62:606-15; J Hematol Oncol 2016, 9:20). D'autre part l'activité anti tumorale de la metformine sur le carcinome hépatocellulaire est décrite par Cauchy et al., 2016, vol. 131 (1), 27-36.

L'iméglimine est une tetrahydrotriazine, premier représentant d'une nouvelle classe chimique d'agents antidiabétiques oraux, les Glimines.

A la différence de la metformine, l'iméglimine n'inhibe pas le complexe I mitochondrial. WO 2010/066901 décrit les propriétés d'activateur de l'AMPK et d'inhibiteur de mTOR de l'iméglimine.

L'iméglimine agit simultanément sur les trois principaux organes directement impliqués dans l'homéostasie du glucose : le foie, les muscles et le pancréas. L'iméglimine inhibe la production hépatique de glucose. Il améliore la sensibilité des organes au glucose et à l'insuline. Il restaure la secrétion d'insuline en réponse au glucose.

Chez le rat diabétique STZ, après 35 jours de traitement, l'iméglimine diminue significativement la glycémie à jeun et l'HbA1c pour des doses de 50 et 100 mg/kg/j. Ces effets sont similaires à ceux de la metformine à 50 mg/kg/j. (Cell Death Discovery 2016 ; 2 : 15072 ; J Diabètes Metab 2011, 2 : 4).

Sur sept études de Phase 1 menées, exposant un total de 216 sujets à l'iméglimine, le produit a montré une excellente tolérance et sécurité d'emploi, avec un nombre comparable d'effets indésirables entre les sujets ayant reçu un placebo et ceux ayant reçu l'iméglimine.

Sur sept études de Phase 2 menées, exposant 611 patients à l'iméglimine, l'efficacité du produit a été évaluée sur des paramètres glycémiques et sur certains paramètres non glycémiques. De même, la tolérance et la sécurité d'emploi ont été évaluées. L'iméglimine a montré une excellente tolérance et une sécurité d'emploi similaires au placebo.

Deux études cliniques de Phase 2a démontrent que l'iméglimine à 1500 mg, administré 2 fois par jour, améliore l'hémoglobine A1c et la glycémie à jeun de façon similaire à la metformine à 850 mg, administrée 2 fois par jour. Ces études montrent un profil de tolérance de l'iméglimine supérieur à celui de la metformine chez les patients diabétiques. En Europe, aux Etats-Unis et au Japon, l'iméglimine est en fin de Phase 2 du développement clinique dans le diabète de type 2. (www.poxel.com/Poxel-Imeglimin-fact-sheet_fr.pdf; Drugs R D 2015, 15 :227-32).

L'iméglimine est protégé par la famille de brevets issue de WO2001/0155122 (déposant Lipha).

Le brevet couvre une formule de Markush incluant l'iméglimine, ainsi qu'un procédé de préparation, les compositions pharmaceutiques et l'utilisation dans le traitement des pathologies associées au syndrome de résistance à l'insuline, plus particulièrement le diabète, les pathologies causées par la formation de produits de glycosylation, la dyslipidémie, l'obésité, l'hypertension artérielle, les rétinopathies et les neuropathies, les complications rénales, l'athérosclérose, l'angiopathie, la maladie d'Alzheimer, les maladies neurodégénératives, la sénilité.

Le demandeur a présentement découvert que l'iméglimine possède de remarquables propriétés pharmacologiques dans le cancer, inattendues, plus particulièrement dans le carcinome hépatocellulaire.

Dans les tests effectués, l'iméglimine a été comparé à la metformine, l'antidiabétique de référence.

L'iméglimine démontre, *in vitro,* un potentiel inhibiteur de prolifération cellulaire deux à quatre fois supérieur à celui de la metformine sur deux lignées de cellules tumorales humaines de cancer primitif du foie (HepG2 et HuH7).

L'iméglimine démontre en outre, *in vivo,* sur des modèles de xénogreffes en sous - cutanée de la lignée HepG2 sur des souris femelles âgées de 6 semaines de fond génétique BALB/c nude (Harlan), un effet inhibiteur sur la croissance tumorale doses dépendantes, non significativement différent du sorafénib, supérieur à la metformine. En outre, l'iméglimine potentialise l'activité du sorafénib, traitement médical de référence du carcinome hépatocellulaire, de façon supérieure à celle de la metformine associée au sorafénib.

L'iméglimine trouve ainsi une application thérapeutique tout à fait intéressante dans la prévention et/ou le traitement du cancer, plus particulièrement dans le carcinome hépatocellulaire.

L'efficacité antitumorale de l'iméglimine associée à son excellente tolérance répond à un réel besoin médical non satisfait dans le traitement des cancers.

L'invention concerne l' iméglimine pour son utilisation dans des compositions pharmaceutiques destinées au traitement curatif et/ou préventif du carcinome hépatocellulaire, seul ou en association avec des anticancéreux tels que les inhibiteurs de kinases, et plus particulièrement sorafénib et régorafénib, les immune checkpoint inhibiteurs, et plus particulièrement le nivolumab. Par ailleurs, l'administration en association peut être sous la forme d'une co-administration simultanée ou successive de deux compositions pharmaceutiques séparées contenant chacune l'un des principes actifs (association libre), ou sous la forme de l'administration d'une association fixe des deux principes actifs au sein de la même composition pharmaceutique.

Les compositions pharmaceutiques seront présentées sous des formes convenant aux administrations par voie orale, parentérale, transcutanée, nasale, rectale, perlinguale, et notamment sous forme de préparations injectables, comprimés sublinguaux, glossettes, gélules, capsules, tablettes, suppositoires, crèmes, pommades, gels dermiques, etc...

Les compositions pharmaceutiques correspondantes peuvent permettre la libération instantanée ou différée de principe actif.

Outre l'iméglimine, les compositions pharmaceutiques selon l'invention contiennent un ou plusieurs excipients ou véhicules choisis parmi des diluants, des lubrifiants, des liants, des agents de désintégration, des absorbants, des colorants, des édulcorants, etc...

A titre d'exemple et de manière non limitative, on peut citer :
- pour les diluants : le lactose, le dextrose, le sucrose, le mannitol, le sorbitol, la cellulose, la glycérine,
- pour les lubrifiants : la silice, le talc, l'acide stéarique et les sels de magnésium et de calcium, le polyéthylène glycol,
- pour les liants : le silicate d'aluminium et de magnésium, l'amidon, la gélatine, la tragacanthe, la méthylcellulose, la carboxyméthylcellulose de sodium et la polyvinylpyrrolidone,
- pour les désintégrants : l'agar, l'acide alginique et son sel de sodium, les mélanges effervescents.

La posologie utile varie selon le sexe, l'âge et le poids du patient, la voie d'administration, la nature de l'affection et des traitements éventuellement associés et s'échelonne entre 25 mg et 6 g d' iméglimine par 24 heures, par exemple entre 500 mg à 4 g par jour.

De manière préférentielle, la dose journalière de l'iméglimine sera de 3 g par jour.

Les exemples suivants illustrent l'invention.

### ETUDE PHARMACOLOGIQUE

**EXEMPLE 1** : Effet antiprolifératif *in vitro* de l'iméglimine comparé à celui de la metformine sur 2 modèles de carcinomes hépatocellulaires (CHC) humains. Détermination de l'IC50 (Concentration Inhibitrice médiane) par le test de prolifération cellulaire MTS Promega^{™} (CellTiter 96^{™} AQueous Nonradioactive Cell Proliferation Assay Kit).

### A) Protocole:

Différentes concentrations en mM (0, 0.1, 0.25, 0.5, 1.0, 2.5, 5.0, 7.5, 10, 25, 50, 100) de l'iméglimine et de la metformine ont été testées *in vitro* sur deux lignées cellulaires humaines de CHC, HepG2 et HuH7, sur une période de 6 jours.

### B) Résultats:

L'effet inhibiteur de l'iméglimine sur la prolifération cellulaire des lignées HepG2 et HuH7 est de 2 à 4 fois plus important que celui de la metformine pour les concentrations de 2.5 à 25 mM. L'effet de l' iméglimine est nettement plus marqué sur la lignée HuH7.

L'effet inhibiteur augmente avec le temps pour l'iméglimine.

Les valeurs d'IC50 de la metformine sont plus élevées que celles de l'iméglimine, d'au moins un facteur 2. Ce ratio augmente avec le temps de traitement, jusqu'à 6 pour HuH7.

**EXEMPLE 2** : Activité antitumorale *in vivo* de l'iméglimine comparée à celle de la metformine, seuls ou associés au sorafenib, sur des modèles de xénogreffes en sous-cutanée de la lignée de CHC HepG2 sur des souris femelles âgées de 6 semaines de fond génétique BALB/c nude (Harlan).

### A) Protocole:

Deux millions de cellules de la lignée HepG2 sont injectées dans le flanc gauche de chacune des souris dans un volume de 100*µ*l (50*µ*lPBS-50*µ*l matrigel). Les souris développant une tumeur palpable sont randomisées en 7 groupes de traitement de 14 souris selon le design suivant : placebo, iméglimine 75 mg/kg/j; iméglimine 150 mg/kg/j , metformine 75 mg/kg/j, sorafenib 40mg/kg/j, iméglimine 75mg/kg/j + sorafénib 40 mg/kg/j, metformine 75 mg/kg/j + sorafenib 40 mg/kg/j. La metformine et l'iméglimine sont administrés dans l'eau de boisson, le sorafenib par gavage oral. Le traitement débute le jour de la randomisation.

Les doses de metformine de 75 mg/kg/j et celle de l'iméglimine de 150 mg/kg/j utilisées dans cette étude *in vivo* chez l'animal correspondent aux doses équipotentes dans le diabète.

Les traitements ont été donnés pendant 63 jours. Les courbes d'évolution des volumes des tumeurs ainsi que celles du poids des animaux ont été analysées pendant cette période. L'aspect des tumeurs et des organes a fait l'objet d'une analyse anatomo-pathologique macroscopique.

### B) Résultats

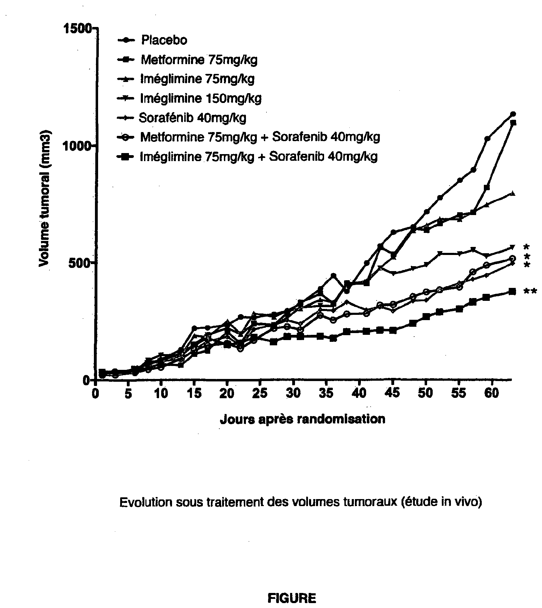

L'iméglimine, la metformine et le sorafénib ralentissent la croissance en volume des tumeurs greffées chez les souris en comparaison au lot témoin placebo (voir FIGURE 1/1).

Pour des doses équipotentes, l'effet sur le volume tumoral de l'iméglimine 150 mg/kg/j est significativement supérieur à celui de la metformine 75 mg/kg/j comparé au placebo.

L'efficacité de l'iméglimine augmente avec le doublement des doses de 75 mg/kg/j à 150 mg/kg/j (effet-dose).

Le traitement par l'iméglimine 150 mg/kg/j n'a pas d'effet significativement différent du sorafénib 40mg/kg/j.

Un effet additionnel sur le volume est noté quand l'iméglimine à 75 mg/kg/j est associé au sorafénib à 40 mg/kg/j. En outre, cette activité est très nettement supérieure à celle de la metformine à 75 mg/kg/j associée au sorafénib à 40 mg/kg/j.

D'un point de vue observationnel, au moment du sacrifice des souris, on remarque que si le sorafénib 40 mg/kg/j seul exerce ses propriétés anti-angiogéniques, la combinaison iméglimine 75 mg/kg/j plus sorafénib 40 mg/kg/j amplifie les effets anti-angiogéniques du sorafénib.

Le traitement par l'iméglimine ne s'est pas avéré toxique, le poids moyen des animaux n'a pas varié au cours des 63 jours de traitement.

### EXEMPLE 3 : Compositions pharmaceutiques

Formulation 1:
iméglimine: 1000 mg
cellulose microcristalline: 150 mg
croscarmellose: 25 mg
polyvinylpyrrolidone: 45 mg
stéarate de magnésium: 10 mg
Eudragit(R): 25 mg

Formulation 2:
iméglimine: 750 mg
sorafénib: 200 mg
cellulose microcristalline: 110 mg
croscarmellose: 21 mg
polyvinylpyrrolidone: 30 mg
stéarate de magnésium: 10,5 mg
Opadry(R): 20 mg

## Revendications

1. Iméglimine destiné à être utilisé à la prévention et/ou au traitement du carcinome hépatocellulaire.

2. Compositions pharmaceutiques contenant l'iméglimine en combinaison avec un ou plusieurs excipients ou véhicules inertes, non toxiques et pharmaceutiquement acceptables, destinées à être utilisées dans la prévention et/ou le traitement du carcinome hépatocellulaire.

3. Compositions pharmaceutiques contenant l'iméglimine et un deuxième principe actif utilisé dans le traitement du carcinome hépatocellulaire en combinaison avec un ou plusieurs excipients ou véhicules inertes, non toxiques et pharmaceutiquement acceptables.

4. Compositions pharmaceutiques selon la revendication 3 où le deuxième principe actif est le sorafenib.

5. Compositions pharmaceutiques selon la revendication 3 ou 4 destinées à être utilisées dans la prévention et/ou le traitement du carcinome hépatocellulaire.

6. Association de l'iméglimine et du sorafenib destinée à être utilisée dans la prévention et/ou le traitement du carcinome hépatocellulaire.

## Patentansprüche

1. Imeglimin zur Verwendung bei der Prävention und/oder Behandlung von hepatozellulärem Karzinom.

2. Pharmazeutische Zusammensetzungen mit Imeglimin in Kombination mit einem oder mehreren inerten, nicht-toxischen and pharmazeutisch annehmbaren Exzipienten oder Trägern zur Verwendung bei der Prävention und/oder Behandlung von hepatozellulärem Karzinom.

3. Pharmazeutische Zusammensetzungen mit Imeglimin und einem zweiten aktiven Inhaltsstoff, der bei der Behandlung von hepatozellulärem Karzinom verwendet wird, in Kombination mit einem oder mehreren inerten, nicht-toxischen and pharmazeutisch annehmbaren Exzipienten oder Trägern.

4. Pharmazeutische Zusammensetzungen nach Anspruch 3, worin der zweite aktive Inhaltsstoff Sorafenib ist.

5. Pharmazeutische Zusammensetzungen nach Anspruch 3 oder 4, zur Verwendung bei der Prävention und/oder Behandlung von hepatozellulärem Karzinom.

6. Assoziierung von Imeglimin und Sorafenib zur Verwendung bei der Prävention und/oder Behandlung von hepatozellulärem Karzinom.

## Claims

1. Imeglimin to use in the prevention and/or treatment of hepatocellular carcinoma.

2. Pharmaceutical compositions containing imeglimin in combination with one or more inert, non-toxic and pharmaceutically acceptable excipients or vehicles to use in the prevention and/or treatment of hepatocellular carcinoma.

3. Pharmaceutical compositions containing imeglimin and a second active ingredient used in the treatment of hepatocellular carcinoma in combination with one or more inert, non-toxic and pharmaceutically acceptable excipients or vehicles.

4. Pharmaceutical compositions according the claim 3, wherein the second active ingredient is sorafenib.

5. Pharmaceutical compositions according to claims 3 or 4, to use in the prevention and/or treatment of hepatocellular carcinoma.

6. Association of imeglimin and of sorafenib to use in the prevention and/or treatment of hepatocellular carcinoma.
